# EUROPÄISCHE PATENTSCHRIFT

(11) **EP 2 088 917 B1**
(45) Veröffentlichungstag und Bekanntmachung des Hinweises auf die Patenterteilung: **14.09.2011**
(21) Anmeldenummer: 08734720.9
(22) Anmeldetag: 20.03.2008
(51) Int. Cl.: A61B 3/00, A61B 19/00, A61F 9/00

(54) **DIAPHANOSKOPSTAB**
DIAPHANOSCOPE ROD
TIGE DE DIAPHONOSCOPE

(30) Priorität: 23.03.2007 DE 102007014703
(43) Veröffentlichungstag der Anmeldung: 19.08.2009
(73) Patentinhaber: Dieter Mann GmbH, 63814 Mainaschaff (DE)
(72) Erfinder: FOERSTER, Michael, 12203 Berlin (DE); BECHRAKIS, Nikolaos, 12203 Berlin (DE); MANN, Dieter, 63839 Kleinwallstadt (DE)
(74) Vertreter: Prüfer & Partner GbR European Patent Attorneys
(86) Internationale Anmeldenummer: PCT/EP2008/002294
(87) Internationale Veröffentlichungsnummer: WO 2008/116609

(56) Entgegenhaltungen:
- EP-A- 0 432 692
- EP-A- 0 651 981
- DE-A1- 4 302 614
- SU-A3- 1 780 502
- US-A- 2 660 732
- US-A- 5 335 648

## Beschreibung

Die vorliegende Erfindung bezieht sich auf einen Diaphanoskopstab.

Bei dem Verfahren der Diaphanoskopie wird das Auge durch eine direkte, auf das Auge gesetzte Lichtquelle durchleuchtet. Es dient dazu, lichtabsorbierende Strukturen im Augeninneren wie Tumore, Blutungen oder Fremdkörper, die bei der Diaphanoskopie Verschattungen verursachen, zu erkennen.

SU 1780502 A3 beschreibt eine Diaphanoskopievorrichtung für Zentralabschnitte des Augengrundes. Ein Lichtleiter weist eine Arbeitsspitze auf, die Z-förmig ist und in die orbitale Lidspalte eingeführt wird.

US 5,335,648 beschreibt ein Instrument zur Beobachtung des Augeninneren, welches als Lichtleiter eine einzelne Lichtleitfaser oder ein Lichtfaserbündel verwendet.

EP 0 651 981 A1 beschreibt eine Beleuchtungssonde zum seitlichen Einstechen in den Augapfel bei der Ophthalmoskopie. Die Sonde enthält eine Lichtleitfaser, deren austrittseitiges Ende mit einer Einrichtung zum Vergrößern des Raumwinkels des abgestrahlten Lichts und damit der beleuchteten Fläche versehen ist.

DE 43 02 614 A1 offenbart eine Beleuchtungseinrichtung für die Diaphanoskopie der Haut *in vivo.* Insbesondere sind eine Lichtquelle und ein flexibler Lichtleiter mit einem lichtleitenden Medium verbunden, das wie ein Trokar ausgebildet ist und an seinem Lichtaustrittsende spitz angeschliffen ist. Die Vorrichtung wird in die Haut eingestochen und in der Unterhaut parallel zur Hautoberfläche vorgeschoben.

US 2,660,732 beschreibt ein künstliches Auge mit einem Implantat, das für die Einführung in die Augenhöhle geeignet gestaltet ist und Verbindungsmittel zur Ankopplung an den Bewegungsmuskel des Auges aufweist.

EP 0 432 692 A1 beschreibt eine Clip-Vorrichtung zum Abklemmen von Blutgefäßen.

Ein bekannter Diaphanoskopstab besteht aus einem Metallrohr, in das Licht leitende Fasern eingebettet sind. Der Lichtstab entwirft ein punktförmiges Licht, das ophthalmoskopisch zu sehen ist. Es wird hierbei üblicherweise ein binokularer, indirekter Augenspiegel verwendet. Der Lichtpunkt ist allerdings zu klein, um die Beurteilung der Lage eines Tantalumclips zum

Tumor vornehmen zu können. Außerdem ist die bereitgestellte Lichtintensität für eine fundierte Diagnose nicht ausreichend.

Die Lichtleitung erfolgt üblicherweise über Glasfaserkabel. Das Grundelement aller faseroptischen Bauteile ist die Lichtfaser. Sie besteht aus einem hochbrechenden Glaskern und einem niedrigbrechenden Glasmantel. Lichtstrahlen, die an der Stirnseite in die Fasern eintreten, werden im Kern durch Totalreflexion an der Grenzfläche Kern/Mantel weitergeleitet und folgen allen Biegungen der Faser, um am Ende wieder auszutreten. Die wichtigsten Kenngrößen von Lichtleitfasern sind die Numerische Apertur, die optische Durchlässigkeit und der Faserdurchmesser.

Es ist die Aufgabe der vorliegenden Erfindung einen Diaphanoskopstab mit verbesserter Lichtleistung bereitzustellen.

Die Aufgabe, einen verbesserten Diaphanoskopstab bereitzustellen, wird durch den Erfindungsgegenstand gemäß Patentanspruch 1 gelöst. Weiterbildungen der Erfindung sind in den Unteransprüchen angegeben.

Der Diaphanoskopstab gemäß der Erfindung hat außerdem den Vorteil, dass er die Kontrolle der Lage eines außen am Auge aufgenähten Tantalumclips zu einem sich im Augeninnern befindenden Tumor ermöglicht. Dies bestimmt auch die Form des Diaphanoskopstabs. Die Kontrolle der Lage ist notwendig, da sie später die Koordinaten für die Bestrahlung des Tumors bestimmt. Die Lichtleitung des Diaphanoskopstabs basiert auf Totalreflexion.

Weitere Merkmale und Zweckmäßigkeiten der Erfindung ergeben sich aus der Beschreibung von Ausführungsbeispielen anhand der beigefügten Zeichnungen. Von den Figuren zeigt:

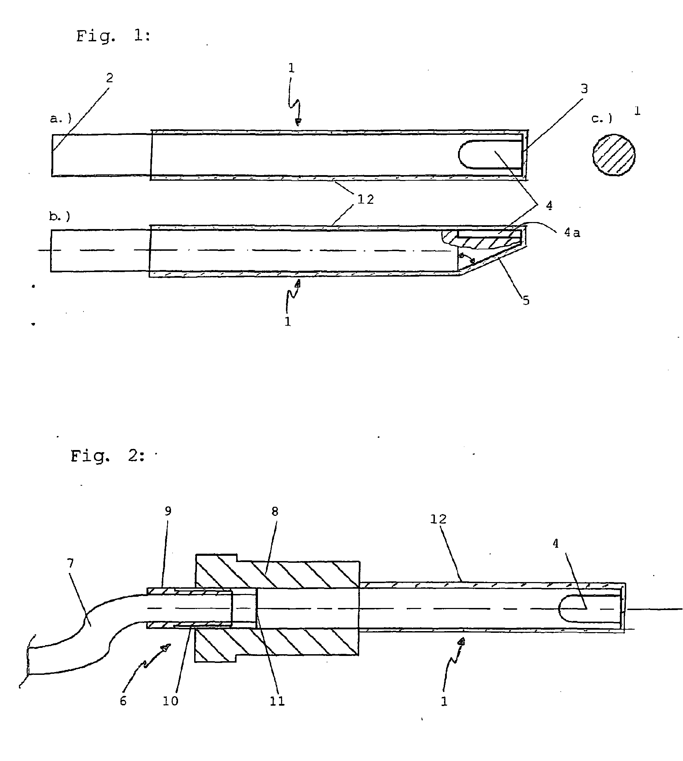

Fig. 1a.) eine Draufsicht auf den Diaphanoskopstab;
Fig. 1b.) einen Längsschnitt durch den Diaphanoskopstab;
Fig. 1c.) einen Schnitt durch den Diaphanoskopstab senkrecht zur Stabachse;
Fig. 2 das teilgeschnittene Diaphanoskop mit Lichtzuführungsvorrichtung.

Der Diaphanoskopstab umfasst einen Stab 1, mit einem ersten Ende 2 und einem zweiten Ende 3, wobei an dem ersten Ende 2 eine Lichtzuführungsvorrichtung 6 vorgesehen ist und an dem zweiten Ende 3 eine Aussparung 4 für z.B. einen Tantalumclip und eine Lichtumlenkvorrichtung 5 vorgesehen ist.

Der Stab 1 weist vorzugsweise einen kreisförmigen oder auch Vierkant-Querschnitt auf und kann jede Länge haben, die der Operateur wünscht. Er besteht aus einem transluzenten Material, beispielsweise aus Glas oder Kunststoff. Es können alle transparenten und transluzenten Kunststoffe bzw. Glasarten verwendet werden. Das hier verwendete Material ist ein optisches Glas, das weitestgehend frei von Einschlüssen ist und ein großes Spektrum an Wellenlängen durchlässt. Das Glas wird auf den entsprechenden Durchmesserstrang gezogen und weist dadurch eine sehr gute Oberflächenqualität auf und hält auch hohen Belastungen stand. Die Wahl des Durchmessers des Stabs 1 kann je nach Anwendung variieren, um mehr Stabilität zu erhalten, bzw. mehr Licht an die Spitze zu bringen.

Der Stab 1 ist an seinem ersten Ende 2 rechtwinklig geschnitten. Der Schnitt kann einfach poliert, geschliffen oder auch optisch poliert sein. Das zweite Ende 3 des Stabes weist die Aussparung 4 auf in die ein Tantalumclip hinein passt. Die Aussparung 4 ist vorzugsweise U-förmig ausgebildet, ähnlich einer zum zweiten Ende 3 offenen Passfedernut. Der Grund der Aussparung 4 ist eben ausgebildet, so dass der Tantalumclip plan aufliegen kann. Die Aussparung 4 kann durch Pressen oder Schleifen auf verschiedene Weise angeformt sein. Falls notwenig, kann die Oberfläche in diesem Bereich nachträglich poliert werden, um eine maximale Transmission zu erreichen. Alle scharfen Kanten, die zum Beispiel durch Schleifen der Aussparung 4 entstehen, sind vorzugsweise verrundet. Dies kann mechanisch erfolgen, durch Polieren oder durch Feuerpolieren und Verwärmen der Kanten und Grate.

Auf der gegenüber liegenden Seite der Aussparung 4, ist die Lichtumlenkvorrichtung 5 vorgesehen, die in diesem Ausführungsbeispiel als Prisma ausgebildet ist. Hierfür ist der Stab 1 an seinem zweiten Ende 3 angeschrägt. Die Ebene in der die Schräge liegt und eine Ebene 4a in der der Grund der Aussparung liegt schließen einen Winkel ein und sind gegeneinander nicht verkippt. Der Winkel des Prismas, also der innere Winkel zwischen der Schräge und der Senkrechten zur Stabachse, ist so gewählt, dass er eine gleichmäßige Ausleuchtung des gewünschten Bereichs gewährleistet. Der Winkel des Prismas hängt auch von der Länge der Aussparung 4 ab, da das zur Stabmitte hin liegende Ende der Aussparung, also der unterste Punkt des U's und das zur Stabmitte hin liegende Ende der Schräge vorzugsweise auf gleicher Höhe in Bezug zur Stabmitte liegen. Der Winkel der Lichtumlenkvorrichtung, bzw. der Winkel des Prismas von ca. 70° hat sich als optimal herausgestellt. Der Winkel ist steil genug, um das Licht im 90° Winkel zur Stabachse direkt in das Auge hinein zu lenken. Ist der Winkel zu steil, ist der Stab an der Spitze zu dick und kann nicht in die Augenhöhle eingeführt werden.

Der Stab könnte auch vollständig oder teilweise mit Sperrfiltern für Wellenlängen, die für das Auge schädlich sind, beschichtet sein oder eine komplette Außenbeschichtung in Form einer Bedampfung oder Beschichtung mit einer Reflexionsschicht oder einer Kunststoff- oder Metallumhüllung 12, um die Lichtleitung weiter zu erhöhen, aufweisen. Bei der Beschichtung muss darauf geachtet werden, dass sie während der Dampfsterilisation des Stabs keinen Schaden nimmt.

Die Lichtzuführungsvorrichtung 6 umfasst ein Kaltlicht-Glasfaserkabel 7, eine erste Halterung 8, eine zweite Halterung 9, die in diesem Ausführungsbeispiel durch ein Gewinde 10 miteinander verbunden sind. An der Verbindungsstelle 11 tritt das Licht aus dem Kaltlicht-Glasfaserkabel 7 in den Stab 1 ein.

Im Vergleich zum Stand der Technik tritt das Licht nicht koaxial aus dem Stab aus, sondern im Winkel von 90 Grad zur Stabachse. Darüber hinaus ist die bereitgestellte Lichtintensität bei der vorliegenden Erfindung höher als beim Stand der Technik.

Im Betrieb wird der Stab direkt auf den Augapfel aufgesetzt, nachdem ein Tantalumclip aufgenäht wurde. Das für die Kontrolle notwendige Licht gelangt durch die Lichtzuführungsvorrichtung 6 in den Stab 1, wird durch diesen hindurch geleitet, von der Lichtumlenkvorrichtung im 90 Grad Winkel zur Stabachse umgeleitet und tritt in Richtung der Aussparung 4 aus. Um die Bestrahlungsplanung und danach die exakte Positionierung des Patienten während der eigentlichen Bestrahlung zu ermöglichen, muss das Bestrahlungszielvolumen exakt definiert werden. Dies wird durch das Aufnähen von röntgendichten Tantalumclips auf der Augenaußenfläche realisiert. Um die exakte Position der auf der Augenaußenfläche aufgenähten Tantalumclips zum Bestrahlungszielvolumen (Tumor) bestimmen zu könnnen, wird der Diaphanoskopstab mit seiner U-förmigen Aussparung auf den Tantalumclip aufgesetzt und der diaphanoskopische Schatten des Tantalumclips mittels indirekter Ophthalmoskopie identifiziert. Seine exakte Position wird auf einer Weitwinkelaufnahme des Augenhintergrundes mit Bezug zu den Tumorrändern markiert. Die Tantalumclips können in dieser Position verbleiben, da sie in der Regel keine Beschwerden verursachen oder ein Hindernis für andere Maßnahmen darstellen.

## Patentansprüche

1. Diaphanoskopstab zum Aufsetzen auf das Auge zur direkten Durchleuchtung des Auges, umfassend eine Lichtzuführungsvorrichtung (6), und **gekennzeichnet durch** einen Stab (1) mit einem ten Ende (2) und einem zweiten Ende (3), bestehend aus transparentem oder transluzentem Material,
wobei ein Ende des Stabs eine Lichtumlenkvorrichtung (5) aufweist.

2. Diaphanoskopstab nach Anspruch 1, wobei die Lichtumlenkvorrichtung (5) ein Prisma ist.

3. Diaphanoskopstab nach Anspruch 2, **dadurch gekennzeichnet, dass** der Stab (1) aus einem Material gebildet wird, das auf den entsprechenden Durchmesser gezogen worden ist.

4. Diaphanoskopstab nach einem der Ansprüche 2 bis 3, **dadurch gekennzeichnet, dass** der Stab an dem ersten Ende (2) rechtwinklig geschnitten ist.

5. Diaphanoskopstab nach einem der Ansprüche 2 bis 4, **dadurch gekennzeichnet, dass** der Stab an dem zweiten Ende (3) eine Aussparung (4) aufweist, die so ausgeformt ist, dass ein Tantalumclip hinein passt.

6. Diaphanoskopstab nach Anspruch 5, **dadurch gekennzeichnet, dass** der Aussparung (4) gegenüber das Prisma angeordnet ist, wobei der Winkel des Prismas so gewählt ist, dass eine gleichmäßige Ausleuchtung des gewünschten Bereichs gewährleistet ist.

7. Diaphanoskopstab nach einem der Ansprüche 5 bis 6, **dadurch gekennzeichnet, dass** die Aussparung (4) eine U-Form aufweist, wobei das zur Stabmitte hin liegende Ende der Aussparung (4) den untersten Punkt des U's bildet.

8. Diaphanoskopstab nach einem der Ansprüche 5 bis 7, **dadurch gekennzeichnet, dass** die schräge Unterseite des Prismas mit der Senkrechten zur Stabachse einen Winkel von 70° bildet.

9. Diaphanoskopstab nach einem der Ansprüche 5 bis 8, **dadurch gekennzeichnet, dass** das zur Stabmitte hin liegende Ende der Aussparung (4), also der unterste Punkt des U's, und das zur Stabmitte hin liegende Ende der Schräge in gleicher Höhe im Verhältnis zur Stabmitte liegen.

10. Diaphanoskopstab nach einem der Ansprüche 1 bis 9, **dadurch gekennzeichnet, dass** alle scharfen Kanten verrundet sind.

11. Diaphanoskopstab nach einem der Ansprüche 1 bis 10, **dadurch gekennzeichnet, dass** der Stab mit Sperrfiltern für Wellenlängen, die für das Auge schädlich sind, beschichtet ist.

12. Diaphanoskopstab nach einem der Ansprüche 1 bis 11, **dadurch gekennzeichnet, dass** der Stab komplett mit einer Reflexionsschicht bedampft und/oder beschichtet ist und/oder mit einer Kunststoff- oder Metall-Schutzhülle (12) ummantelt ist.

## Claims

1. Diaphanoscope rod for being put onto the eye for a direct transillumination of the eye comprising a light feeding device (6) and being **characterized by** a rod (1) having a first end (2) and a second end (3), said rod consisting of transparent or translucent material,
wherein one end of the rod comprises a light deflecting device (5).

2. Diaphanoscope rod according to claim 1, wherein the light deflecting device (5) is a prism.

3. Diaphanoscope rod according to claim 2, **characterized in that** the rod (1) is formed of a material that has been drawn to the corresponding diameter.

4. Diaphanoscope rod according to one of claims 2 to 3, **characterized in that** the rod is cut in a right angle at the first end (2).

5. Diaphanoscope rod according to one of the claims 2 to 4, **characterized in that** the rod comprises a recess (4) at the second end (3), which recess is formed in such a manner that a tantalum clip fits therein.

6. Diaphanoscope rod according to claim 5, **characterized in that** the prism is arranged opposite to the recess (4), wherein the prism angle is selected such that a uniform illumination of the desired area is ensured.

7. Diaphanoscope rod according to one of claims 5 to 6, **characterized in that** the recess (4) has a U-shape, wherein that end of the recess (4) that is facing the rod center forms the lower-most point of the U.

8. Diaphanoscope rod according to one of claims 5 to 7, **characterized in that** the slanted bottom side of the prism and the perpendicular to the rod axis form an angle of 70°.

9. Diaphanoscope rod according to one of claims 5 to 8, **characterized in that** the end of the recess (4) that faces the rod center, thus the lower-most point of the U, and the end of the slant that faces the rod center lie at the same height with respect to the rod center.

10. Diaphanoscope rod according to one of claims 1 to 9, **characterized in that** all sharp edges are rounded.

11. Diaphanoscope rod according to one of claims 1 to 10, **characterized in that** the rod is coated with blocking filters for wavelengths that are harmful to the eye.

12. Diaphanoscope rod according to one of claims 1 to 11, **characterized in that** the rod is evaporated and/or coated with a reflective layer and/or is sheathed with a plastic protective cover (12) or metal protective cover (12).

## Revendications

1. Tige de diaphanoscope, destinée à être posée sur l'oeil pour examiner directement l'oeil à la lumière, comportant un dispositif d'acheminement de la lumière (6) et **caractérisée par** une tige (1) avec une première extrémité (2) et une seconde extrémité (3), réalisée dans un matériau transparent ou translucide, une extrémité de la tige comportant un dispositif de déviation de la lumière (5).

2. Tige de diaphanoscope selon la revendication 1, dans laquelle le dispositif de déviation de la lumière (5) est un prisme.

3. Tige de diaphanoscope selon la revendication 2, **caractérisée en ce que** la tige (1) est réalisée dans un matériau qui a été étiré jusqu'au diamètre approprié.

4. Tige de diaphanoscope selon l'une quelconque des revendications 2 à 3, **caractérisée en ce que** la tige est coupée à angle droit au niveau de la première extrémité (2).

5. Tige de diaphanoscope selon l'une quelconque des revendications 2 à 4, **caractérisée en ce que** la tige, au niveau de sa seconde extrémité (3), comporte un évidement (4) qui est conformé de manière à pouvoir y ajuster un clip en tantale.

6. Tige de diaphanoscope selon la revendication 5, **caractérisée en ce que** l'évidement (4) est agencé en regard du prisme, l'angle du prisme étant choisi de manière à garantir un éclairage homogène de la zone souhaitée.

7. Tige de diaphanoscope selon l'une quelconque des revendications 5 à 6, **caractérisée en ce que** l'évidement (4) a une forme en U, l'extrémité de l'évidement (4), orientée vers le milieu de la tige, formant le point le plus bas du U.

8. Tige de diaphanoscope selon l'une quelconque des revendications 5 à 7, **caractérisée en ce que** la face inférieure inclinée du prisme forme un angle de 70° avec la perpendiculaire à l'axe de la tige.

9. Tige de diaphanoscope selon l'une quelconque des revendications 5 à 8, **caractérisée en ce que** l'extrémité de l'évidement (4), orientée vers le milieu de la tige, c'est-à-dire le point le plus bas du U, et l'extrémité, orientée vers le milieu de la tige, de la surface inclinée sont situées à la même hauteur par rapport au milieu de la tige.

10. Tige de diaphanoscope selon l'une quelconque des revendications 1 à 9, **caractérisée en ce que** toutes les arêtes vives sont arrondies.

11. Tige de diaphanoscope selon l'une quelconque des revendications 1 à 10, **caractérisée en ce que** la tige est revêtue de filtres d'arrêt pour des longueurs d'ondes qui sont nocives pour l'oeil.

12. Tige de diaphanoscope selon l'une quelconque des revendications 1 à 11, **caractérisée en ce que** la tige est totalement métallisée avec une couche réfléchissante et/ou revêtue de ladite couche et/ou entourée d'une gaine de protection (12) en matière plastique ou en métal.
